# EUROPEAN PATENT SPECIFICATION

(11) **EP 3 007 830 B1**
(45) Date of publication and mention of the grant of the patent: **01.05.2019**
(21) Application number: 14747303.7
(22) Date of filing: 03.06.2014
(51) Int. Cl.: B05B 7/04, A61L 9/14, B05B 1/04, B05B 7/08, B05B 7/24, B05B 15/70, B05B 15/55, B05B 15/555, A61L 2/22

(54) **AN IMPROVED MIST-GENERATING DEVICE**
VERBESSERTE NEBELERZEUGENDE VORRICHTUNG
DISPOSITIF DE GÉNÉRATION DE BROUILLARD AMÉLIORÉ

(30) Priority: 13.06.2013 GB 201310576
(43) Date of publication of application: 20.04.2016
(73) Proprietor: 3M Innovative Properties Company, St. Paul, Minnesota 55144 (US)
(72) Inventor: DOSWELL, David, Huntingdon Cambridgeshire PE28 3YF (GB); FRENCH, James, Huntingdon Cambridgeshire PE29 1SE (GB)
(74) Representative: Murgitroyd & Company
(86) International application number: PCT/EP2014/061433
(87) International publication number: WO 2014/198583

(56) References cited:
- GB-A- 2 095 586
- US-A- 3 749 315
- US-A- 4 275 838

## Description

The present invention is concerned with mist-generation in rooms and other enclosed spaces for the purposes of, for example, disinfection or decontamination. More specifically, the present invention provides an improved mist-generating device and portable mist-generating apparatus.

Twin-fluid mist-generating devices which can generate and spray mists are known. Typically, such atomisers use a high velocity driving fluid, such as compressed air or steam, to apply shear forces to a process liquid (i.e. the liquid which is to be sprayed), thereby atomising the process liquid into small droplets which are sprayed from the device as a dispersed phase within a continuous vapour phase of the driving fluid. Such devices have been used in disinfection and decontamination processes where the small droplet size and reach of the spray provides an improved coverage of surfaces in the space being treated, including non-line of sight surfaces.

When such a device is used in a disinfection or decontamination process, it is necessary to remove any residual chemicals from the device before it can be used again. To remove these chemicals it is necessary to transport the device to an enclosed cleaning location, and run cleaning liquid through the device which is sprayed out as a mist into the cleaning location space. Moving the device to a cleaning location, connecting a supply of cleaning liquid in place of the process liquid, running the cleaning process and then moving the device to another space in need of treatment is time-consuming. Furthermore, simply spraying an uncontrolled amount of cleaning liquid through the device is not an efficient or economical way of cleaning the device.

It is an aim of the present invention to obviate or mitigate one or more of these disadvantages.

US3749315A discloses a centrifugal dispensing device particularly adapted to intermittent application of a selected liquid such as glue, paint and the like. The device includes a disc positioned to rotate in a horizontal plane.

According to a first aspect of the invention there is provided a mist-generating device comprising:
a housing;
a nozzle located within the housing, the nozzle movable between a retracted position inside the housing and a deployed position outside the housing;
a drain chamber defined between the housing and nozzle when the nozzle is in the retracted position; and
a drain passage providing fluid communication away from the drain chamber;.
wherein the nozzle comprises:
   a nozzle inlet connectable to a source of driving fluid, a nozzle outlet, and a nozzle throat intermediate the nozzle inlet and nozzle outlet, the nozzle throat having a cross sectional area which is less than that of both the nozzle inlet and nozzle outlet; and
   a process liquid chamber having an inlet connectable to a source of process liquid and an outlet which opens into the nozzle.

The process liquid chamber outlet may open into the nozzle between the nozzle throat and the nozzle outlet.

The device may further comprise a nozzle head upon which the nozzle is located, the nozzle head having a longitudinal axis, and wherein the nozzle extends in a substantially radial direction relative to the longitudinal axis.

The nozzle head may further comprise:
a driving fluid passage having an inlet connectable to the source of driving fluid and an outlet in fluid communication with the nozzle inlet; and
a process liquid conduit having an inlet connectable to the source of process liquid and an outlet in fluid communication with the process liquid chamber;
wherein the driving fluid passage and process liquid conduit extend longitudinally through the nozzle head,

The process liquid conduit may include a removable filter element.

The nozzle may extend circumferentially about the nozzle head such that the nozzle covers a rotational angle about the longitudinal axis. The rotational angle may be substantially 360 degrees.

The device may further comprise first and second substantially planar opposing surfaces which define the nozzle therebetween, and wherein the process liquid chamber outlet is located on one of the first and second surfaces.

The process liquid chamber and chamber outlet may be substantially annular.

The device may further comprise a drive mechanism for moving the nozzle between the retracted and deployed positions, the mechanism comprising:
a support column slidably mounted in the housing and having the nozzle fixed thereto;
a threaded driving rod having a drive carriage rotatably mounted thereon;
a collar member having a first portion attached to the support column and a second portion attached to the drive carriage such that rotation of the driving rod translates into an axial movement of the drive carriage and support column; and
a drive motor adapted to provide rotational movement of the driving rod.

The drive mechanism may reciprocate the nozzle between the deployed position and an intermediate position adjacent the retracted position, the intermediate position being outside the housing.

The device may further comprise a removable catch tank in fluid communication with the drain passage.

The drain chamber may be an annular chamber extending around the circumference of the nozzle when the nozzle is in the retracted position.

The device may further comprise a gasket arranged around the circumference of the nozzle, wherein when the nozzle is in the retracted position the gasket forms a seal between the nozzle and the housing and partially defines the drain chamber.

The device may further comprise one or more visual indicators which indicate the operational status of the device.

According to a second aspect of the invention there is provided a portable mist-generating apparatus comprising:
a compressor unit including a compressor, a compressor inlet providing driving fluid to the compressor, and a compressor outlet through which compressed driving fluid exits the compressor; and
a spraying unit comprising:
   a mist-generating device in accordance with the first aspect of the invention;
   a process liquid vessel and a cleaning liquid vessel in fluid communication with the nozzle via a liquid supply line;
   a driving fluid inlet having a first end connectable to the compressor outlet and a second end in fluid communication with the nozzle; and
   a first control valve which selectively permits fluid communication of one of the process liquid and cleaning liquid vessels to the liquid supply line.

The spraying unit may further comprise a pneumatic pump and a pump supply line connected to the driving fluid inlet, the pump pumping process or cleaning liquid through the liquid supply line to the nozzle.

The pump supply line may include a bypass line and a second control valve located on the bypass line, whereby driving fluid can selectively bypass the pump so as to purge the liquid supply line and the nozzle.

The compressor unit may further comprise an air filter and/or air dryer intermediate the compressor and the compressor outlet.

The spraying unit may be carried upon the compressor unit when the apparatus is being transported.

The compressor unit may include one or more clamps which secure the spraying unit to the compressor unit during transportation.

According to a third aspect of the invention there is provided a method of operating a mist-generating device having a nozzle located within a housing, the method comprising the steps of:
moving the nozzle to a deployed position outside the housing;
supplying a process liquid to the nozzle;
spraying a mist of process liquid droplets from the nozzle;
stopping the supply of process liquid to the nozzle;
moving the nozzle to a retracted position inside the housing;
supplying a cleaning liquid to the nozzle;
spraying cleaning liquid from the nozzle into a drain chamber defined between the housing and retracted nozzle; and
draining the cleaning liquid away from the drain chamber through a drain passage;
wherein the nozzle has a nozzle inlet, a nozzle outlet and a nozzle throat which has a smaller cross sectional area than both the nozzle inlet and the nozzle outlet, and wherein the step of spraying the mist of process fluid droplets comprise the steps of:
   connecting a source of driving fluid to the nozzle inlet;
   connecting a source of the process liquid to a process liquid outlet which opens into the nozzle; and
   accelerating the driving fluid through the nozzle throat, such that the driving fluid atomises the process liquid entering the nozzle through the process liquid outlet.

The nozzle may extend in a substantially radial direction relative to a longitudinal axis of the device, and the steps of spraying the process and cleaning liquids may comprise spraying the respective liquids radially about the longitudinal axis.

The nozzle may cover a rotational angle of substantially 360 degrees about the longitudinal axis, and the steps of spraying the process and cleaning liquids may comprise spraying the respective liquids over substantially 360 degrees about the longitudinal axis.

The steps of supplying the liquids to the nozzle may comprise pumping the liquids using a pneumatic pump which is driven by driving fluid from the driving fluid source.

The method may further comprise the step of purging the nozzle of process and/or cleaning liquid using driving fluid from the driving fluid source.

The steps of moving the nozzle between the deployed and retracted positions may be effected by a drive motor of a drive mechanism.

The method may further comprise the step of operating the drive mechanism so as to reciprocate the nozzle between the deployed position and an intermediate position adjacent the retracted position, the intermediate position being outside the housing.

The method may further comprise the step of draining the cleaning liquid from the drain chamber to a removable catch tank in fluid communication with the drain passage.

A preferred embodiment of the present invention will now be described, by way of example only, with reference to the following drawings:
Figure 1 is a perspective view of a portable compressor unit forming part of a mist-generating apparatus;
Figure 2 is a perspective view of a portable spraying unit forming part of the mist-generating apparatus;
Figures 3(a)-3(c) are respectively front, top and side views of the mist-generating apparatus;
Figure 4 is a schematic view of the mist-generating apparatus;
Figure 5(a) is a first view of a mist-generating device forming part of the spraying unit;
Figure 5(b) is a section view taken along line A-A shown in Figure 5(a);
Figure 5(c) is a second view of the mist-generating device taken at right angles to the view of Figure 5(a);
Figure 5(d) is a section view taken along line C-C in Figure 5(c);
Figure 6 is a detail view of a nozzle head seen in the section view of Figure 5(b);
Figure 7 is a further detail view of an area of the nozzle head indicated by the box marked "7" in Figure 6; and
Figure 8 is an enlarged view of the nozzle in the nozzle head shown in Figures 6 and 7.

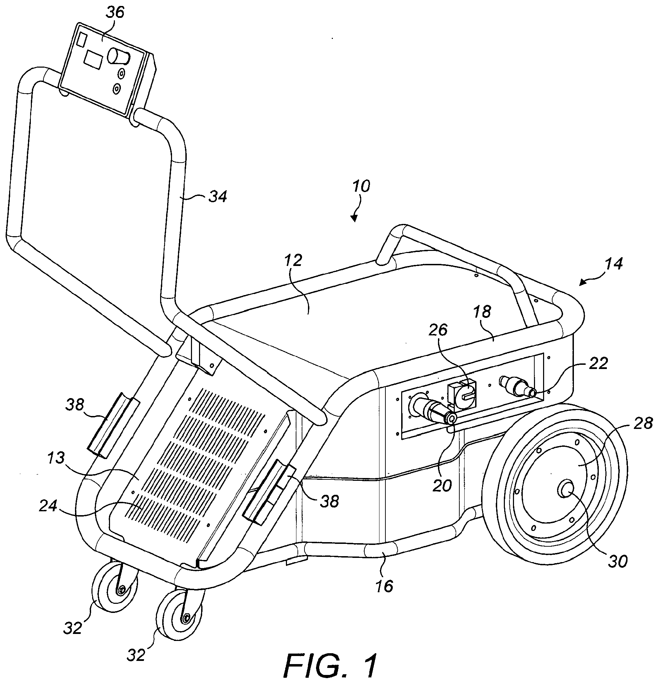

Figure 1 shows a portable compressor unit 10 which forms part of a mist-generating apparatus. The compressor unit 10 comprises a body 12 which is supported by a tubular chassis 14. The chassis 14 comprises a lower section 16 and an upper section 18. The chassis sections 16,18 may be integrally formed with one another or else may be attached at their respective rear ends in a conventional manner by, for example, welding. The body 12 is contained by the chassis sections 16,18 and houses a compressor (not shown in figure 1). Extending from the body 12 are a compressed air outlet port 20 through which air flows from the compressor, and a connector 22 to which power and control cables are connected to take power and control signals from the compressor unit 10 to a spraying unit, which will be described further below. An air inlet grille 24 is also provided at the rear of the body 12 for drawing air into the compressor. Located between the outlet port 20 and connector 22 is a power socket 26 which is connected in use to an electrical power supply to provide power to the compressor unit 10.

The lower chassis section 16 is supported at its front end by a pair of wheels 28, which are rotatably supported on individual axle members 30, or else may be supported by a single axle member extending transversely across the unit 10. A pair of castors 32 are attached at the rear of the lower chassis section 16 in order to assist with the steering of the unit 10.

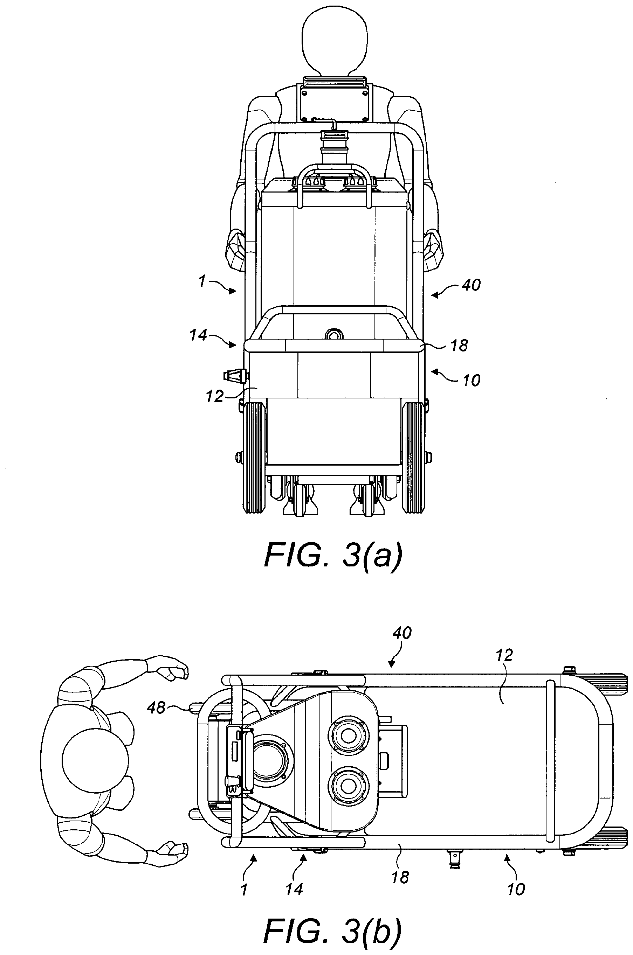

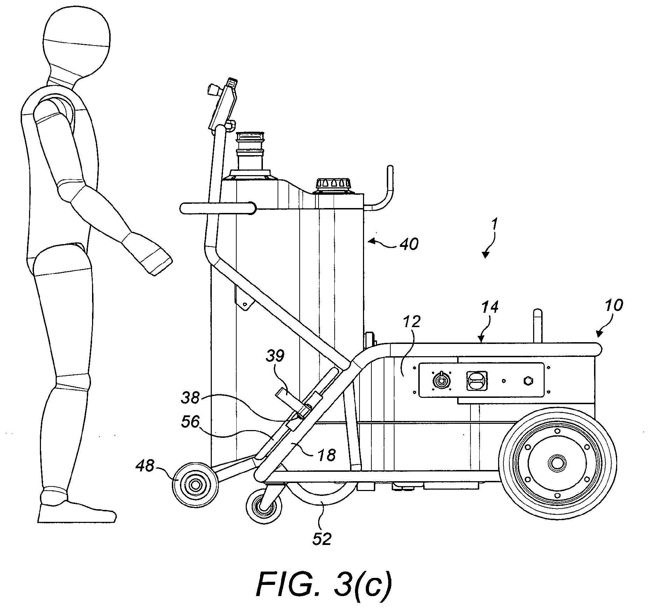

The upper chassis section 18 includes a handle portion 34, which may be integrally formed with the remainder of the upper chassis section 18. A control panel 36 for an operator is attached to the handle portion 34. A lockable clamp 38 is provided on either side of the rear portion of the upper chassis section 18 for securing a portable spraying unit to the compressor unit 10, as will be described in more detail with reference to figure 3.

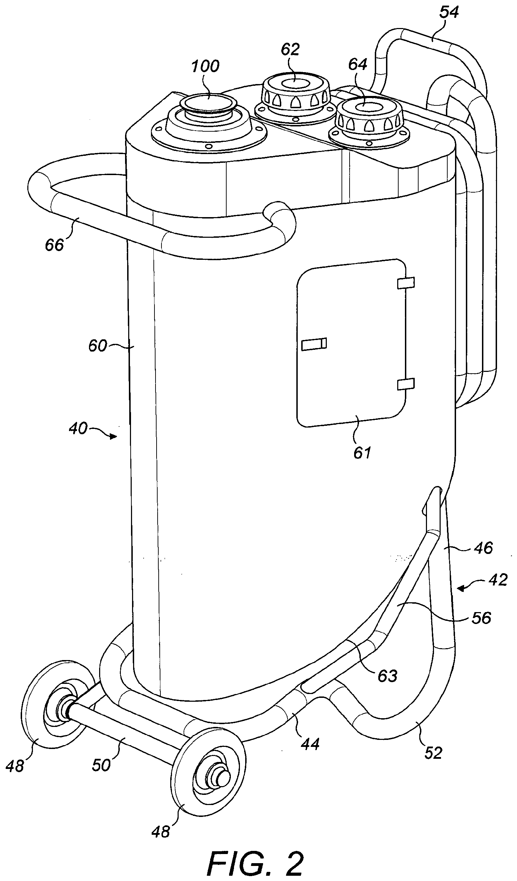

Figure 2 shows a portable spraying unit 40 forming part of a mist-generating apparatus. The spraying unit 40 comprises a tubular chassis 42 having a lower chassis section 44 and an upper chassis section 46. Attached to the rear of the lower chassis section 44 is a pair of wheels 48 rotatably supported on an axle member 50. The front of the lower chassis section 44 includes a pair of support feet 52, with one foot 52 provided on either side of the lower chassis section 44. The upper chassis section 46 extends substantially vertically from the front section of the lower chassis section 44 so as to form a support column for the spraying unit 40. The top portion of the upper chassis section 46 may include a support 54 about which a compressor hose may be stored. Extending between the lower and upper chassis sections 44,46 are a pair of support braces 56, with one brace 56 located on either side of the spraying unit 40.

Mounted upon the chassis 42 is a housing 60. Within the housing 60 are a disinfectant vessel and a cleaning liquid vessel whose respective filler caps 62,64 are located on the top of the housing 60. The housing 60 also contains a door 61 for the removal and emptying of a cleaning fluid catch tank (not shown). Also located within the housing 60 is a mist-generating device which includes a retractable nozzle head 100 which extends from the top of the housing 60. The housing 60 also includes a handle 66, which may form part of the upper chassis section 46.

A mist-generating apparatus 1 made up of the compressor unit 10 and spraying unit 40 is shown in figures 3(a)-3(c). As can be seen, in order to facilitate easy transportation of the apparatus 1 to a disinfection site, the spraying unit 40 is carried upon the compressor unit 10. To place the spraying unit 40 upon the compressor unit 10, the spraying unit 40 is wheeled to the rear of the compressor unit 10 and the feet 52 of the spraying unit 40 are placed in gaps between the compressor body 12 and upper chassis section 18 on either side of the compressor unit (best viewed in figure 1). Once the feet 52 are in the gaps the spraying unit 40 can be pivoted forward over the rear of the compressor chassis 14 such that the wheels 48 of the spraying unit 40 leave the ground and the weight of the spraying unit 40 is then supported by the compressor unit 10. A base 63 of the spray housing 60 and a rear surface 13 of the compressor body are both inclined such that they abut one another when the spraying unit 40 is carried by the compressor unit 10. Once the spraying unit 40 is supported on the compressor unit 10, the braces 56 on the spraying chassis 42 lie upon the clamps 38 of the compressor chassis 14 and the clamps 38 are then closed to lock the two units 10,40 together. Detachable locking members 39 may be employed to lock the clamps 38 in the closed position.

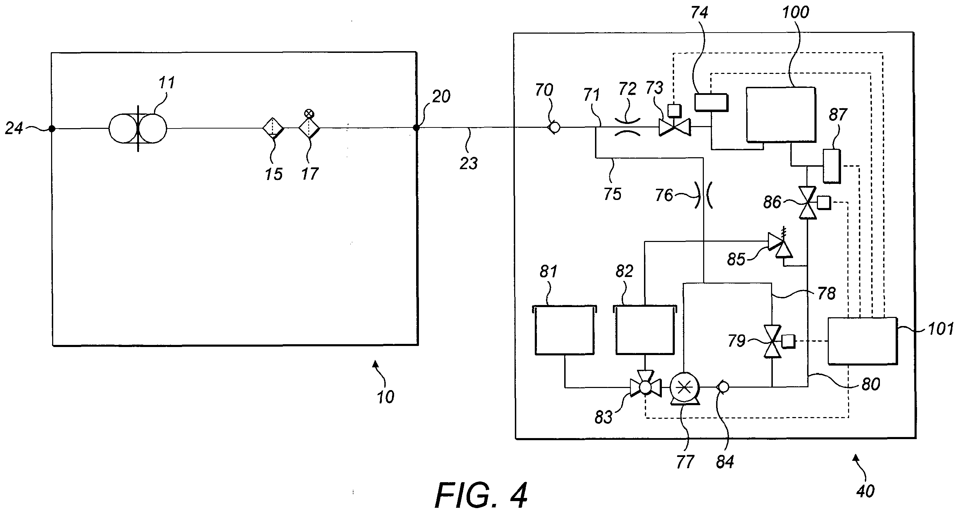

Figure 4 is a schematic view of the mist-generating apparatus formed from the compressor unit 10 and the spraying unit 40. The compressor unit 10 contains a compressor 11 which receives air through the air inlet grille 24. The compressed air passes from the compressor 11 through an air filter 15 and an air dryer 17 to the compressed air outlet 20. An air line 23 on the spraying unit 40 is connected to the air outlet 20 of the compressor unit 10 in order to supply compressed air into the spraying unit 40.

Air entering the spraying unit 40 passes through a non-return valve 70 before branching into a nozzle supply line 71 supplying air to the nozzle head 100, and a pump supply line 75 supplying air to a pneumatic pump 77 which pumps the liquid in the liquid feed section of the system. The nozzle supply line 71 includes a first throttle/regulator 72, a first control valve 73 which controls flow of air to the nozzle head 100, and a pressure sensor 74 which monitors air pressure between the control valve 73 and the nozzle head 100.

The liquid feed section of the system is formed around the liquid supply line 80. At the upstream end of the liquid line 80 are cleaning liquid and disinfectant vessels 81,82. A pressure relief valve 85 is in fluid communication with both the disinfectant vessel 82 and the liquid line 80 downstream of the pump 77. Supply of disinfectant or cleaning liquid into the liquid line 80 is controlled by a second, three-way control valve 83. When the second control valve 83 is open to either of the cleaning or disinfectant vessels 81,82, liquid is drawn into the liquid line 80 by the pneumatic pump 77, and passes through a non-return valve 84. A third control valve 86 immediately upstream of the nozzle head 100 controls flow of the liquid into the nozzle head 100, with the pressure between the control valve 86 and nozzle head 100 being monitored by a further pressure sensor 87.

Air passing though the pump supply line 75 passes through a second throttle/regulator 76 into the pump 77. When the system is to be purged, air may also be drawn off from the pump supply directly to a liquid supply line 80 via a bypass line 78 which is controlled by a fourth control valve 79.

The four control valves 73,79,83,86 and the two pressure sensors 74,87 are in communication with an electronic control unit (ECU) 101, which controls the operation of the mist-generating apparatus. The ECU 101 is also in communication with the control panel 36 located on the handle of the compressor unit 10, thereby allowing the operator to control the system from outside the space being misted.

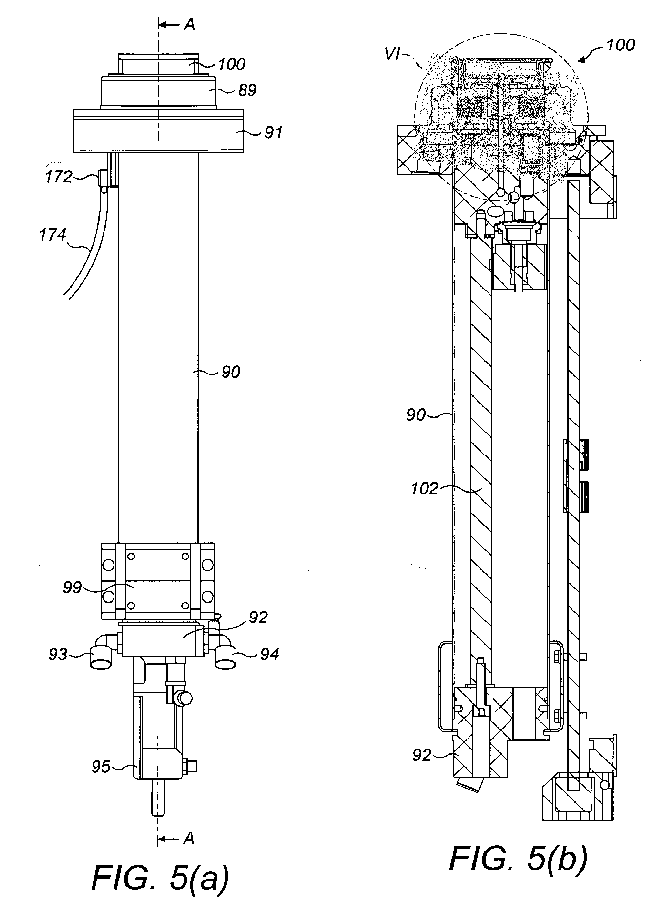

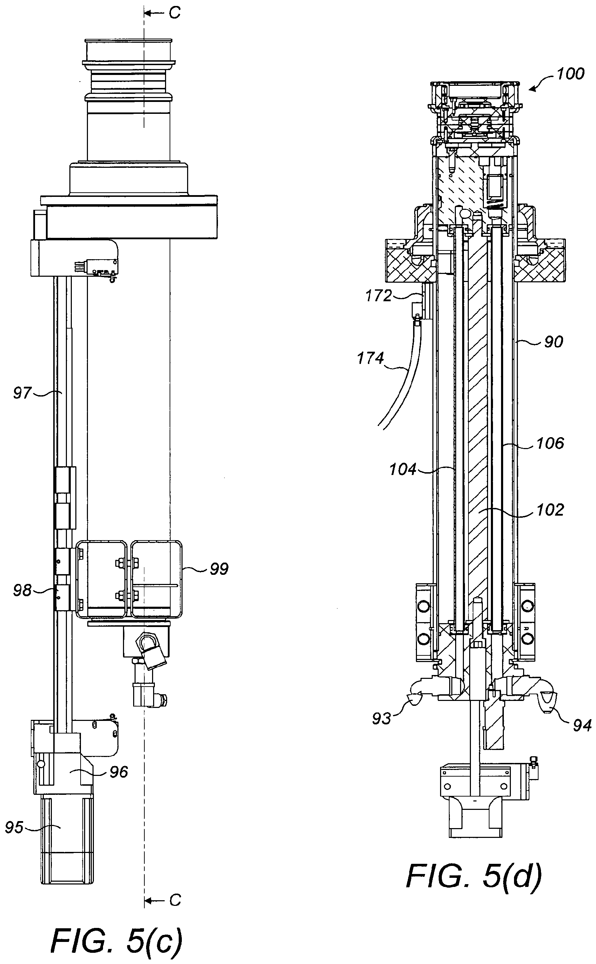

A mist-generating device which is housed within the spraying unit 40 is shown in front and side views in figures 5(a) and 5(c), with the spraying unit omitted for illustrative purposes. The section view of figure 5(b) is taken along line A-A in figure 5(a), whilst the section view of figure 5(d) is taken along line C-C in figure 5(c). Figures 5(a) and 5(b) show the nozzle head in the retracted position, whilst figures 5(b) and 5(d) show the nozzle head in the raised position.

The mist-generating device comprises a support column 90 upon the top of which the nozzle head 100 is located. At the lower end of the column 90 is a manifold 92 which has inlets 93,94 connected in use to the air supply line 71 and liquid supply line 80, respectively. Although not part of the support column 90, figure 5 does also show a guide collar 91 which is fixed to the top of the spraying unit 40 and ensures that the mist-generating device rises and falls in the correct alignment. Fixed atop the collar 91 is a nozzle cover 89 which encloses the nozzle head 100 when in its retracted position.

A drive mechanism is provided for raising and lowering the mist-generating device and nozzle head 100, and is best viewed in figure 5(c). The drive mechanism comprises an electric motor 95 which has a drive shaft 96 connected to a threaded rod, also known as a lead screw, 97 which extends upwards in parallel with the support column 90. A drive carriage 98 is fixed to a drive collar 99 which is in turn fixed to the lower end of the support column 90. The drive carriage 98 is rotatably mounted on the lead screw 97 such that rotation of the lead screw 97 will cause the carriage 98 to move axially along the lead screw 97. As a result, clockwise or counter-clockwise rotation of the lead screw 97 by the electric motor 95 will axially move the carriage 98 along the lead screw 97 and hence cause the column 90 to raise or lower relative to the spraying unit.

As can be seen in the section views of figures 5(b) and 5(d), the support column 90 is hollow and the manifold 92 and nozzle head 100 are secured to one another by a tie bar 102 which extends along the inside of the column 90. An air supply hose 104 and a liquid supply hose 106 are provided either side of the tie bar 102 and connect their respective manifold inlets 93,94 with the nozzle head 100.

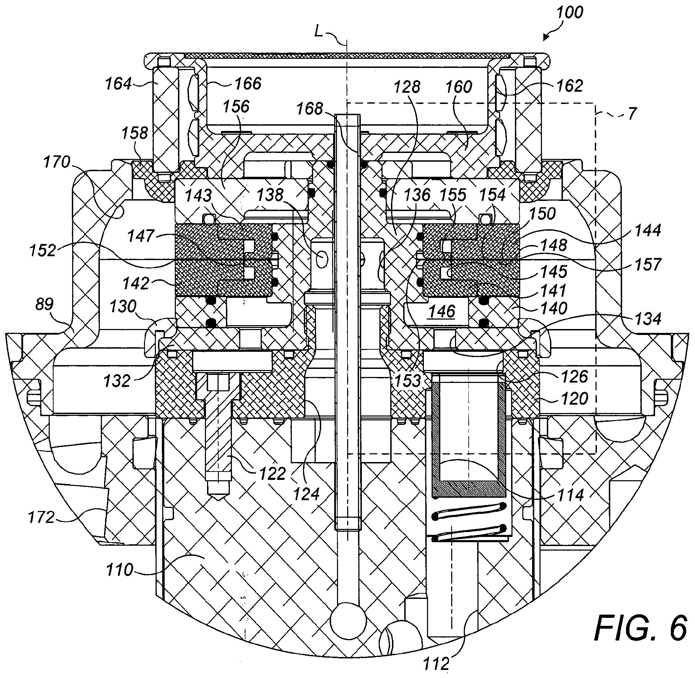

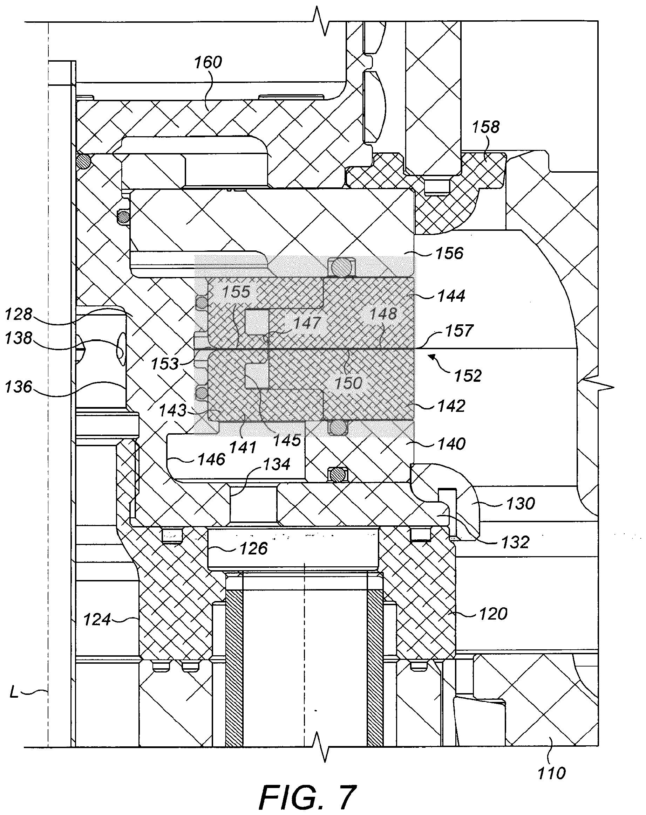

The nozzle head 100 is shown in more detail in the section view of figures 6 and 7, which are enlarged versions of the section shown in figure 5(b), with the area shown in Figure 7 marked by box "7" in figure 6. Figures 6 and 7 show the nozzle head 100 in its retracted position atop the spraying unit 40. The nozzle head 100 is fixed upon a support member 110, which is secured to the manifold 92 by the tie bar 102 described above with reference to figure 5. The support member 110 includes a liquid supply conduit 112 and a gas/air supply conduit (not shown), which are connected to the downstream ends of the supply hoses 104,106. Lying partially within the liquid conduit 112 is a spring-loaded filter element 114, which can be replaced or cleaned upon removal of the nozzle head 100 from the support member 110.

The nozzle head 100 itself is comprised of an annular base member 120 which is fixed by screws 122 onto the support member 110. Defined within the base member 120 are a central air passage 124 which is in fluid communication with the air supply conduit of the support member 110, and an annular liquid supply chamber 126 which is in fluid communication with the liquid supply conduit 112. A nozzle core member 128 is secured to the base member 120 by a locking ring 130. The core member 128 has a radially projecting flange portion 132 adjacent the base member 120, and the flange portion 132 includes a plurality of longitudinally extending passages 134 which communicate with the liquid supply chamber 126 in the base member 120. Extending longitudinally through the centre of the core member 128 is an air distribution chamber 136, which is in fluid communication with the central air passage 124 and has a plurality of air passages 138 extending radially outward from the distribution chamber 136. A first annular spacer 140 is located upon the upper face of the flange portion 132. An annular first inner nozzle member 141 and an annular first outer nozzle member 142 are then placed upon the first spacer 140. An outer surface of the inner nozzle member 141 and an inner surface of the outer nozzle member 142 abut one another and between them define a liquid supply chamber 145. The first spacer 140 and inner and outer nozzle members 141,142 are placed upon the core member 128 before the core member 128 is secured to the base member 120. A plurality of fixing screws (not shown) extend from the bottom side of the core flange 132 through apertures (not shown) in the core flange 132, first spacer 140 and outer nozzle member 142 so as to secure these components to one other. Between them, the core member 128, first spacer 140 and inner nozzle member 141 define an annular liquid supply plenum 146. The plenum 146 is in fluid communication with the liquid supply chamber 145 via passages (not shown) in the inner nozzle member 141.

An annular second inner nozzle member 143 and an annular second outer nozzle member 144 are fixed to a second annular spacer 156 by a plurality of fixing screws (not shown) which extend from the top side of the spacer 156 through apertures (not shown) in the spacer and second outer nozzle member 144. An outer surface of the inner nozzle member 143 is sandwiched between the spacer 156 and an inner surface of the outer nozzle member 144 such that the inner nozzle member 143 is secured to the spacer 156.

The first nozzle members 141,142 and the second nozzle members 143,144 define first and second nozzle surfaces 148,150, respectively. These nozzle surfaces 148,150 are substantially planar and face one another to define a nozzle 152. "Substantially planar" should be understood as meaning that the surfaces are substantially flat, but includes surfaces which may be provided with surface roughing or small detent portions to influence the flow in the nozzle if desired. The nozzle surfaces 148,150 are held at a predetermined distance from one another by the respective screw fixtures in the first and second nozzle members 141,142,143,144 in order to maintain a nozzle gap 154 having a desired width.

An annular external gasket 158 is placed about the circumference of the second spacer 156. A cap member 160 is then secured to the second spacer 156 by bolts (not shown) to fix all of the nozzle head components in place. The nozzle cap 160 includes a number of coloured light-emitting diodes (LEDs) 162 in order to show the operational status of the nozzle. The cap 160 includes a wiring chamber 166 and a wiring conduit 168 extends longitudinally through the nozzle head 100 for housing the wiring (not shown) of the LEDs. An annular lens 164 lies between the cap 160 and gasket 158 in order to protect the LEDs 162. As can be seen in figure 6, when the nozzle head 100 is in the retracted position the outer rim of the gasket 158 seals against an inner surface of the nozzle cover 89, thus creating an external drain chamber 170 between the nozzle head 100 and nozzle cover 89. The drain chamber 170 has an external drain port 172 to which a drain line 174 is connected in order to draw fluid away from the drain chamber 170 to the cleaning fluid catch tank located behind door 61 in the spraying unit housing 60 (see figure 2). The catch tank is also provided with a pressure relief valve for releasing compressed air from the tank whilst catching all of the cleaning fluid.

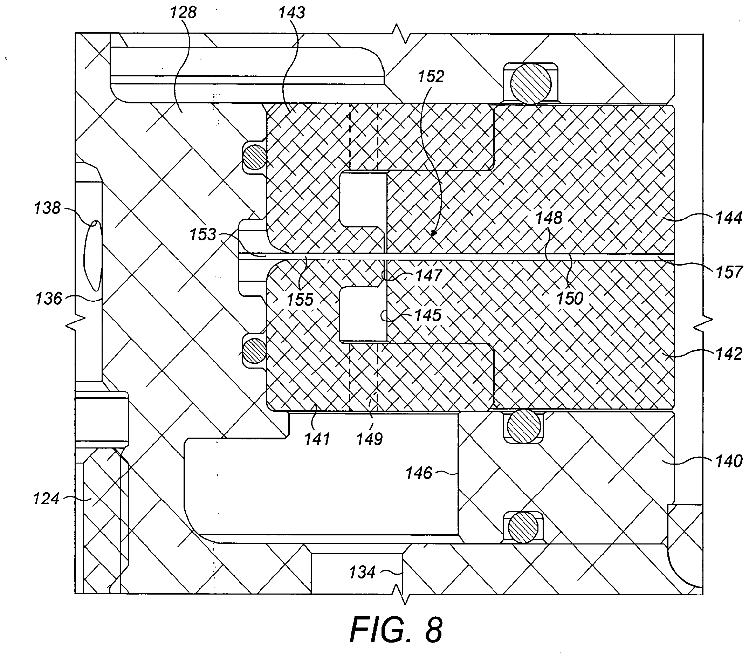

The completed nozzle 152 which results when all of the nozzle head components are assembled is shown in figure 8. The nozzle 152 comprises a nozzle inlet 153 in fluid communication with the radial air passages 138, a nozzle outlet 157 radially outward of the nozzle inlet 153, and a nozzle throat 155 intermediate the nozzle inlet 153 and nozzle outlet 157. The nozzle throat 155 has a cross sectional area which is less than that of both the nozzle inlet 153 and nozzle outlet 157. A liquid outlet 147 of the liquid supply chamber 145 opens into the nozzle 152 upon the first nozzle surface 148 intermediate the nozzle inlet 153 and nozzle outlet 157. The liquid outlet 147 may be downstream of the nozzle throat 155.

The nozzle head 100 has a longitudinal axis L. The central air passage 124 and liquid conduit 112 extend longitudinally through the nozzle head 100, and the nozzle 152 extends in a substantially radial direction relative to the longitudinal axis L.

The operation of the mist-generating apparatus will now be described, with the apparatus being used for a disinfection process in this illustrative example. Initially, the apparatus is wheeled to a location close to the space to be disinfected in the state shown in figure 3. The compressor unit 10 is then unlocked and dismounted from the spraying unit 40. An umbilical hose containing the power and control lines and the air line 23 is then run from the spraying unit 40 and the respective lines are connected to the compressed air outlet 20 and connector 22 on the compressor unit 10. The spraying unit 40 can then be wheeled into the space to be disinfected leaving the compressor unit 10 and system controls outside the space and, using the control panel 36 on the compressor unit 10, the operator can then begin the disinfection process.

Firstly, when instructed by the operator the control panel will communicate with a transceiver connected to the electric motor 95 of the mist-generating device within the spraying unit 40. The transceiver will instruct the motor 95 to rotate the lead screw 97 so as to raise the nozzle column 90 and nozzle head 100. The operator will then instruct the control panel 36 to start up the compressor 11 and compressed air will enter the spraying unit 40 and the nozzle and pump supply lines 71,75. The control panel will also signal the ECU 101 to open the first control valve to allow compressed air to flow to the nozzle head 100 as well as the pump 77. At the same time, the ECU 101 is instructed to set the second control valve 83 so that disinfectant can flow from the disinfectant vessel 82 and the disinfectant is drawn down the liquid supply line 80 by the pump 77. Flow of the compressed air and disinfectant into the nozzle head 100 is then controlled by the ECU 101 signalling the first and third control valves 73,86 in response to signals received from the pressure sensors 74,87.

Referring again to figures 6 and 8, when the compressed air arrives at the nozzle head 100, it enters via the central passage 124 and distribution chamber 136 before passing to the nozzle inlet 153 via air passages 138. At the same time, disinfectant is entering the nozzle head 100 via liquid supply conduit 112, supply chamber 126, and longitudinal passages 134 before arriving in the plenum 146. From the plenum 146, the disinfectant enters the liquid supply chamber 145 via interconnecting passages 149 which are located in the first inner nozzle member 141. The disinfectant is then sprayed from the liquid supply outlet 147 of the liquid supply chamber 145 as a thin, annular film into the nozzle 152. The compressed air is accelerated as it flows through the nozzle throat 155 and this accelerated, preferably supersonic, flow of air comes into contact with the film of disinfectant exiting the liquid outlet 147. As the air and disinfectant come into contact an energy transfer takes place between the two, primarily as a result of mass and momentum transfer between the high velocity air and the relatively low velocity disinfectant. This energy transfer imparts a shearing force on the disinfectant, leading to the atomisation thereof. Atomisation is used herein to refer to the break up of the disinfectant into small droplets. This atomisation leads to the creation of a dispersed droplet-vapour flow regime spraying from the nozzle outlet 157 over a preferred spray angle of 360 degrees about a longitudinal axis of the nozzle head 100. A dispersed droplet-vapour flow regime is used herein to describe a mist comprising a dispersed phase of disinfectant process liquid droplets in a continuous vapour phase of compressed air driving fluid.

The atomisation of the disinfectant is achieved using primary and secondary break-up mechanisms. The primary mechanism is the high shear force applied to the disinfectant by the compressed air, which forms ligaments at the boundary surface of the liquid disinfectant. These ligaments are stripped from the surface and atomised into droplets. Two secondary break-up mechanisms further atomise the droplets produced by the primary break-up. These secondary mechanisms are a further shear force caused by the remaining differential between the relative velocities of the compressed air and disinfectant within the nozzle 152, and the turbulent eddy break-up of the disinfectant caused by the turbulent flow of the expanding air radially outward from the nozzle throat 155. The disinfectant mist generated by the apparatus preferably has a majority of droplets whose diameters are between 1 and 10 microns, and most preferably between 1 and 5 microns. The nozzle outlet 157 extends around the entire perimeter of the nozzle head 100 and the mist sprayed from the apparatus may exit the apparatus at a spray angle of substantially 360 degrees about the transport fluid passage 128.

There may be two groups of LEDs 162 provided in the cap 160, with one group of red LEDs and one group of green LEDs. During the disinfection operation, the ECU 101 may illuminate the red LEDs to show that the device is active and that it is not safe to enter the space being misted. The ECU 101 is pre-programmed to leave a dwell time after disinfection is completed before switching off the red LEDs and illuminating the green LEDs to show that the space is now safe to enter.

Once the disinfection operation is complete, the system can be switched over to a cleaning mode. Firstly, the second control valve 83 is closed to all liquid flow so that no further disinfectant is drawn out of the disinfectant vessel 82 and the compressor 11 is turned off such that no further compressed air is supplied to the spraying unit 40. The transceiver will then instruct the motor 95 to rotate the lead screw 97 in the opposite direction to before so as to retract the nozzle column 90 and nozzle head 100 into the nozzle cover 89. Once the nozzle head 100 is fully retracted the external gasket 158 on the nozzle head 100 seals against an inner surface of the nozzle cover 89, thus creating the external drain chamber 170 between the nozzle head 100 and nozzle cover 89. A drain line is connected to the drain port 172 in order to drain fluid from the drain chamber 170.

The operator will then instruct the control panel 36 to re-start the compressor 11 and compressed air will once again enter the spraying unit 40 and the nozzle and pump supply lines 71,75. The control panel will also signal the ECU 101 to open the first control valve to allow compressed air to flow to the nozzle head 100 as well as the pump 77. At the same time, the ECU 101 is instructed to open the second control valve 83 to cleaning liquid flow from the cleaning liquid vessel 81. The cleaning liquid is drawn down the liquid supply line 80 by the pump 77 and sprayed out of the nozzle in the same manner as described above when in the disinfecting mode. Alternatively, the first control valve 73 may remain closed so that all of the compressed air is sent to the pump 77, and the cleaning liquid is simply pumped through the system and out of the nozzle 152.

Because the nozzle head 100 is retracted, all of the cleaning liquid issuing from the nozzle outlet 157 is captured within the drain chamber 170 and drawn out of the mist-generating device via the drain port 172. The gasket 158 ensures that no cleaning liquid is sprayed outside of the mist-generating device.

Once the cleaning operation has been completed, the second control valve 83 is instructed to close and cut off the supply of cleaning liquid from the cleaning liquid vessel 81. The fourth control valve 79 in the pump bypass line 78 is then opened so that the compressed air in the pump supply line 75 is all diverted into the liquid supply line 80 in order to purge any remnants of disinfectant or cleaning liquid from the liquid supply line 80 and nozzle into the catch tank via the drain chamber 170. As described above, the catch tank has a pressure relief valve which will release the compressed air from the tank whilst retaining the captured cleaning fluid. Once this is complete, the ECU 101 will again shut down the compressor 11 and instruct the various components of the system to reset to their initial positions in readiness for the next disinfecting operation.

The present invention provides a mist-generating device which can be subjected to a cleaning operation without any of the cleaning liquid being emitted from the device into the atmosphere surrounding the device. It also provides an apparatus with integral cleaning function so that the apparatus can be switched over to a cleaning mode without any work needing to be carried out by an operator. In addition, with the apparatus comprising separate compressor and spraying units the operator can monitor and control the operation of the apparatus via the compressor unit from a safe location outside the space in which misting is to take place. However, allowing the spraying unit to be mounted on the compressor unit during transportation means that the system can still be transported by a single operator.

By using a pneumatic pump powered by air from the compressor, the system is to a certain extent controlled by the operation of the compressor. If the compressor is not operating and connected to the spraying unit the pump cannot draw fluid down the liquid supply line to the nozzle. This simplifies the controls needed at the operator interface/display unit.

The process liquid vessel may include a spray head connected to the cleaning liquid vessel, such that cleaning liquid is sprayed into the process liquid vessel during the cleaning mode. Alternatively, or in addition, the second control valve may have a setting whereby cleaning liquid can enter both the process liquid vessel and liquid supply line during the cleaning mode.

The deployment and retraction mechanism for the nozzle head and support column may move the nozzle head to deployed position and then retract the nozzle head into its retracted position once the misting operation has been completed. Alternatively, the mechanism may continuously raise and lower the nozzle head during the misting operation. In such a case, the mechanism would be set up so that the nozzle head would not fully retract into the nozzle cover during the lowering phase.

The driving fluid used in the preferred example is compressed air, but alternative driving fluids, such as steam or nitrogen, may also be used. The "compressor unit" may not in fact contain a compressor but may instead simply contain a pressurised driving fluid source such as, for example, a pressurised gas bottle containing nitrogen. References herein to "air passage" or "air chamber" should therefore be interpreted as passages and chambers for conducting any suitable driving fluid and not just compressed air.

Whilst the description of the operation of the present invention related to a disinfection process, the present invention is not limited to such a process. Other mist-generating processes such as decontamination may also be conducted using the present invention.

A linear, twin-fluid nozzle may be incorporated in the present invention in place of the radial, 360 degree nozzle described herein.

The nozzle head may be deployed and retracted manually, although the preferred method is using the powered drive mechanism described above.

Whilst LEDs are the preferred visual indicators, other indicating means may be incorporated in the present invention such as, for example, conventional coloured light bulbs or an liquid crystal display (LCD) message board. An aural indicator, such as a series of beeps or chirps emitted from a loudspeaker connected to the system, may be used alongside or in place of the visual indicators.

The liquids may be gravity-fed in the mist-generating apparatus rather than using a pump. Where a pump is used, an electrical pump may be incorporated in the present invention in place of the described pneumatic pump.

These and other modifications and improvements may be incorporated without departing from the scope of the present invention.

## Claims

1. A mist-generating device comprising:
a housing (60);
a nozzle (152) located within the housing (60), the nozzle movable between a retracted position inside the housing (60) and a deployed position outside the housing (60);
a drain chamber (170) defined between the housing (60) and nozzle (152) when the nozzle is in the retracted position; and
a drain passage providing fluid communication away from the drain chamber (170);
**characterised in that** the nozzle (152) comprises:
a nozzle inlet (153) connectable to a source of driving fluid, a nozzle outlet (157), and a nozzle throat (155) intermediate the nozzle inlet (153) and nozzle outlet (157), the nozzle throat (155) having a cross sectional area which is less than that of both the nozzle inlet (153) and nozzle outlet (157); and
a process liquid chamber (145) having an inlet connectable to a source of process liquid and an outlet which opens into the nozzle (152).

2. The device of claim 1, wherein the process liquid chamber outlet opens into the nozzle (152) between the nozzle throat (155) and the nozzle outlet (157).

3. The device of either preceding claim, further comprising a nozzle head (100) upon which the nozzle (152) is located, the nozzle head (100) having a longitudinal axis (L), and wherein the nozzle (152) extends in a substantially radial direction relative to the longitudinal axis (L).

4. The device of claim 3, wherein the nozzle (152) extends circumferentially about the nozzle head (100) such that the nozzle covers a rotational angle about the longitudinal axis (L).

5. The device of claim 4, wherein the nozzle (152) covers a rotational angle of substantially 360 degrees about the longitudinal axis (L).

6. The device of any of claims 3 to 5, further comprising first and second substantially planar opposing surfaces (148,150) which define the nozzle (152) therebetween, and wherein the process liquid chamber (145) outlet is located on one of the first and second surfaces.

7. The device of claim 6, wherein the process liquid chamber (145) and chamber outlet are substantially annular.

8. The device of any preceding claim, further comprising a drive mechanism for moving the nozzle (152) between the retracted and deployed positions, the mechanism comprising:
a support column (90) slidably mounted in the housing (60) and having the nozzle (152) fixed thereto;
a threaded driving rod (97) having a drive carriage (98) rotatably mounted thereon;
a collar member (99) having a first portion attached to the support column (90) and a second portion attached to the drive carriage (98) such that rotation of the driving rod (97) translates into an axial movement of the drive carriage (98) and support column (90); and
a drive motor (95) adapted to provide rotational movement of the driving rod (97).

9. The device of claim 8, wherein the drive mechanism reciprocates the nozzle (152) between the deployed position and an intermediate position adjacent the retracted position, the intermediate position being outside the housing (60).

10. The device of any preceding claim, further comprising a removable catch tank in fluid communication with the drain passage.

11. The device of any preceding claim, wherein the drain chamber (170) is an annular chamber extending around the circumference of the nozzle (152) when the nozzle is in the retracted position.

12. The device of claim 11 further comprising a gasket (158) arranged around the circumference of the nozzle (152), wherein when the nozzle is in the retracted position the gasket (158) forms a seal between the nozzle and the housing (60) and partially defines the drain chamber (170).

13. A portable mist-generating apparatus comprising:
a compressor unit (10) including a compressor, a compressor inlet providing driving fluid to the compressor, and a compressor outlet through which compressed driving fluid exits the compressor; and
a spraying unit (40) comprising:
a mist-generating device in accordance with any of claims 1 to 13;
a process liquid vessel (82) and a cleaning liquid vessel (81) in fluid communication with the nozzle (152) via a liquid supply line (80);
a driving fluid inlet having a first end connectable to the compressor outlet and a second end in fluid communication with the nozzle (152); and
a first control valve (73) which selectively connects one of the process liquid and cleaning liquid vessels (82,81) with the liquid supply line (80).

14. The apparatus of claim 13, wherein the spraying unit (40) further comprises a pneumatic pump (77) and a pump supply line (75) connected to the driving fluid inlet, the pump driven by driving fluid to pump process or cleaning liquid through the liquid supply line (80) to the nozzle.

15. The apparatus of claim 14, wherein the pump supply line (75) includes a bypass line (78) and a second control valve (83) located on the bypass line (78), whereby driving fluid can selectively bypass the pump (77) so as to purge the liquid supply line (80) and the nozzle (152).

16. The apparatus of any of claims 13 to 15, wherein the spraying unit (40) is carried upon the compressor unit (10) when the apparatus is being transported.

17. The apparatus of claim 16, wherein the compressor unit (10) includes one or more clamps (38) which secure the spraying unit (40) to the compressor unit (10) during transportation.

18. A method of operating a mist-generating device having a nozzle (152) located within a housing (60), the method comprising the steps of:
moving the nozzle (152) to a deployed position outside the housing (60);
supplying a process liquid to the nozzle;
spraying a mist of process liquid droplets from the nozzle;
stopping the supply of process liquid to the nozzle;
moving the nozzle to a retracted position inside the housing (60);
supplying a cleaning liquid to the nozzle;
spraying cleaning liquid from the nozzle into a drain chamber (170) defined between the housing (60) and retracted nozzle; and
draining the cleaning liquid away from the drain chamber (170) through a drain passage;
**characterised in that** the nozzle (152) has a nozzle inlet (153), a nozzle outlet (157) and a nozzle throat (155) which has a smaller cross sectional area than both the nozzle inlet (153) and the nozzle outlet (157), and wherein the step of spraying the mist of process fluid droplets comprises the steps of:
connecting a source of driving fluid to the nozzle inlet (153);
connecting a source of the process liquid to a process liquid outlet which opens into the nozzle (152); and
accelerating the driving fluid through the nozzle throat (155), such that the driving fluid atomises the process liquid entering the nozzle through the process liquid outlet.

19. The method of claim 18, wherein the nozzle (152) extends in a substantially radial direction relative to a longitudinal axis (L) of the device, and the steps of spraying the process and cleaning liquids comprise spraying the respective liquids radially about the longitudinal axis (L).

20. The method of claim 19 wherein the nozzle (152) covers a rotational angle of substantially 360 degrees about the longitudinal axis (L), and the steps of spraying the process and cleaning liquids comprise spraying the respective liquids over substantially 360 degrees about the longitudinal axis (L).

21. The method of any of claims 18 to 20, wherein the steps of supplying the liquids to the nozzle (152) comprise pumping the liquids using a pneumatic pump (77) which is driven by driving fluid from the driving fluid source.

22. The method of any of claims 18 to 21, further comprising the step of purging the nozzle of process and/or cleaning liquid using driving fluid from the driving fluid source.

23. The method of any of claims 18 to 22, wherein the steps of moving the nozzle (152) between the deployed and retracted positions are effected by a drive motor (95) of a drive mechanism.

24. The method of claim 23, further comprising the step of operating the drive mechanism so as to reciprocate the nozzle (152) between the deployed position and an intermediate position adjacent the retracted position, the intermediate position being outside the housing (60).

25. The method of any of claims 18 to 24, further comprising the step of draining the cleaning liquid from the drain chamber (170) to a removable catch tank in fluid communication with the drain passage.

## Patentansprüche

1. Dunsterzeugungsvorrichtung, umfassend:
ein Gehäuse (60);
eine innerhalb des Gehäuses (60) angeordnete Düse (152), wobei die Düse zwischen einer eingefahrenen Position innerhalb des Gehäuses (60) und einer ausgefahrenen Position außerhalb des Gehäuses (60) bewegbar ist;
eine Ablaufkammer (170), die zwischen dem Gehäuse (60) und der Düse (152) definiert ist, wenn sich die Düse in der eingefahrenen Position befindet; und
einen Ablaufdurchgang, der eine Fluidverbindung weg von der Ablaufkammer (170) bereitstellt;
**dadurch gekennzeichnet, dass** die Düse (152) Folgendes umfasst:
einen Düseneinlass (153), der mit einer Quelle von Antriebsfluid verbunden werden kann, einen Düsenauslass (157) und einen Düsenhals (155) zwischen dem Düseneinlass (153) und dem Düsenauslass (157), wobei der Düsenhals (155) einen Querschnittsbereich aufweist, der kleiner ist als der des Düseneinlasses (153) und des Düsenauslasses (157); und
eine Prozessflüssigkeitskammer (145) mit einem Einlass, der mit einer Quelle von Prozessflüssigkeit verbunden werden kann, und einem Auslass, der sich in die Düse (152) öffnet.

2. Vorrichtung gemäß Anspruch 1, wobei der Prozessflüssigkeitskammerauslass in die Düse (152) zwischen dem Düsenhals (155) und dem Düsenauslass (157) mündet.

3. Vorrichtung gemäß einem der vorstehenden Ansprüche, ferner umfassend einen Düsenkopf (100), auf dem sich die Düse (152) befindet, wobei der Düsenkopf (100) eine Längsachse (L) aufweist und wobei sich die Düse (152) in einer im Wesentlichen radialen Richtung relativ zur Längsachse (L) erstreckt.

4. Vorrichtung gemäß Anspruch 3, wobei die Düse (152) in Umfangsrichtung um den Düsenkopf (100) verläuft, sodass die Düse einen Drehwinkel um die Längsachse (L) abdeckt.

5. Vorrichtung gemäß Anspruch 4, wobei die Düse (152) einen Drehwinkel von im Wesentlichen 360 Grad um die Längsachse (L) abdeckt.

6. Vorrichtung gemäß einem der Ansprüche 3 bis 5, ferner umfassend eine erste und zweite im Wesentlichen ebene gegenüberliegende Fläche (148, 150), die dazwischen die Düse (152) definieren, und wobei sich der Auslass der Prozessflüssigkeitskammer (145) auf einer der ersten und zweiten Oberfläche befindet.

7. Vorrichtung gemäß Anspruch 6, wobei die Prozessflüssigkeitskammer (145) und der Kammerauslass im Wesentlichen ringförmig sind.

8. Vorrichtung gemäß einem der vorstehenden Ansprüche, ferner umfassend einen Antriebsmechanismus zum Bewegen der Düse (152) zwischen der eingefahrenen und der ausgefahrenen Position, wobei der Mechanismus Folgendes umfasst:
eine Stützsäule (90), die verschiebbar in dem Gehäuse (60) montiert ist und die die Düse (152) daran befestigt aufweist;
eine mit Gewinde versehene Antriebsstange (97), auf der ein Antriebsschlitten (98) drehbar montiert ist;
ein Manschettenelement (99) mit einem ersten Abschnitt, der an der Stützsäule (90) angebracht ist, und einem zweiten Abschnitt, der derart an dem Antriebsschlitten (98) angebracht ist, dass sich die Drehung der Antriebsstange (97) in eine axiale Bewegung des Antriebsschlittens (98) und der Stützsäule (90) umwandelt; und
einen Antriebsmotor (95), der so angepasst ist, dass er eine Drehbewegung der Antriebsstange (97) bereitstellt.

9. Vorrichtung gemäß Anspruch 8, wobei der Antriebsmechanismus die Düse (152) zwischen der ausgefahrenen Position und einer Zwischenposition benachbart zur eingefahrenen Position hin- und herbewegt, wobei die Zwischenposition außerhalb des Gehäuses (60) liegt.

10. Vorrichtung gemäß einem der vorstehenden Ansprüche, die ferner einen abnehmbaren Auffangbehälter in Fluidverbindung mit dem Ablaufdurchgang umfasst.

11. Vorrichtung gemäß einem der vorstehenden Ansprüche, wobei die Ablaufkammer (170) eine ringförmige Kammer ist, die sich um den Umfang der Düse (152) erstreckt, wenn sich die Düse in der eingefahrenen Position befindet.

12. Vorrichtung gemäß Anspruch 11, ferner umfassend eine um den Umfang der Düse (152) angeordnete Dichtung (158), wobei, wenn sich die Düse in der eingefahrenen Position befindet, die Dichtung (158) einen Verschluss zwischen der Düse und dem Gehäuse (60) bildet und teilweise die Ablaufkammer (170) definiert.

13. Tragbare Dunsterzeugungseinrichtung, umfassend:
eine Kompressoreinheit (10) mit einem Kompressor, einem Kompressoreinlass, der dem Kompressor Antriebsfluid bereitstellt, und einen Kompressorauslass, durch den komprimiertes Antriebsfluid aus dem Kompressor austritt; und
eine Sprüheinheit (40), umfassend:
eine Dunsterzeugungsvorrichtung gemäß einem der Ansprüche 1 bis 13;
einen Prozessflüssigkeitsbehälter (82) und einen Reinigungsflüssigkeitsbehälter (81) in Fluidverbindung mit der Düse (152) über eine Flüssigkeitszuleitung (80);
einen Antriebsfluideinlass mit einem ersten Ende, das mit dem Kompressorauslass verbunden werden kann, und einem zweiten Ende in Fluidverbindung mit der Düse (152); und
ein erstes Steuerventil (73), das einen der Prozessflüssigkeits- und Reinigungsflüssigkeitsbehälter (82, 81) wahlweise mit der Flüssigkeitszuleitung (80) verbindet.

14. Einrichtung gemäß Anspruch 13, wobei die Sprüheinheit (40) ferner eine pneumatische Pumpe (77) und eine Pumpenzuleitung (75) umfasst, die mit dem Antriebsfluideinlass verbunden ist, wobei die Pumpe durch Antriebsfluid angetrieben wird, um Prozess- oder Reinigungsflüssigkeit durch die Flüssigkeitszuleitung (80) zu der Düse zu pumpen.

15. Einrichtung gemäß Anspruch 14, wobei die Pumpenzuleitung (75) eine Bypassleitung (78) und ein auf der Bypassleitung (78) befindliches zweites Steuerventil (83) einschließt, wobei Antriebsfluid die Pumpe (77) selektiv umgehen kann, um die Flüssigkeitszuleitung (80) und die Düse (152) zu spülen.

16. Einrichtung gemäß einem der Ansprüche 13 bis 15, wobei die Sprüheinheit (40) auf der Kompressoreinheit (10) getragen wird, wenn die Einrichtung transportiert wird.

17. Einrichtung gemäß Anspruch 16, wobei die Kompressoreinheit (10) eine oder mehrere Klemmen (38) einschließt, die die Sprüheinheit (40) während des Transports an der Kompressoreinheit (10) sichern.

18. Verfahren zum Betreiben einer Dunsterzeugungsvorrichtung mit einer Düse (152), die sich in einem Gehäuse (60) befindet, wobei das Verfahren die folgenden Schritte umfasst:
Bewegen der Düse (152) in eine ausgefahrene Position außerhalb des Gehäuses (60);
Zuführen einer Prozessflüssigkeit zu der Düse;
Sprühen eines Dunsts von Prozessflüssigkeitströpfchen aus der Düse;
Stoppen der Zufuhr von Prozessflüssigkeit zu der Düse;
Bewegen der Düse in eine eingefahrene Position innerhalb des Gehäuses (60);
Zuführen einer Reinigungsflüssigkeit zu der Düse;
Sprühen von Reinigungsflüssigkeit aus der Düse in eine zwischen dem Gehäuse (60) und der eingefahrenen Düse definierte Ablaufkammer (170); und
Ablassen der Reinigungsflüssigkeit weg von der Ablaufkammer (170) durch einen Ablaufdurchgang;
**dadurch gekennzeichnet, dass** die Düse (152) einen Düseneinlass (153), einen Düsenauslass (157) und einen Düsenhals (155) aufweist, der eine kleinere Querschnittsfläche als sowohl der Düseneinlass (153) als auch der Düsenauslass (157) aufweist, und wobei der Schritt des Sprühens des Dunsts von Prozessfluidtröpfchen die folgenden Schritte umfasst:
Verbinden einer Quelle von Antriebsfluid mit dem Düseneinlass (153);
Verbinden einer Quelle der Prozessflüssigkeit mit einem Prozessflüssigkeitsauslass, der in die Düse (152) mündet; und
Beschleunigung des Antriebfluids durch den Düsenhals (155), sodass das Antriebsfluid die Prozessflüssigkeit zerstäubt, die durch den Prozessflüssigkeitsauslass in die Düse eintritt.

19. Verfahren gemäß Anspruch 18, wobei sich die Düse (152) in einer im Wesentlichen radialen Richtung relativ zu einer Längsachse (L) der Vorrichtung erstreckt und die Schritte des Sprühens der Prozess- und Reinigungsflüssigkeiten das Sprühen der jeweiligen Flüssigkeiten radial um die Längsachse (L) umfassen.

20. Verfahren gemäß Anspruch 19, wobei die Düse (152) einen Drehwinkel von im Wesentlichen 360 Grad um die Längsachse (L) abdeckt und die Schritte des Sprühens der Prozess- und Reinigungsflüssigkeiten das Sprühen der jeweiligen Flüssigkeiten über im Wesentlichen 360 Grad um die Längsachse (L) umfassen.

21. Verfahren gemäß einem der Ansprüche 18 bis 20, wobei die Schritte des Zuführens der Flüssigkeiten zur Düse (152) das Pumpen der Flüssigkeiten unter Verwendung einer pneumatischen Pumpe (77) umfassen, die durch Antriebsfluid von der Antriebsfluidquelle angetrieben wird.

22. Verfahren gemäß einem der Ansprüche 18 bis 21, ferner umfassend den Schritt des Spülens der Düse von Prozess- und/oder Reinigungsflüssigkeit unter Verwendung von Antriebsfluid von der Antriebsfluidquelle.

23. Verfahren gemäß einem der Ansprüche 18 bis 22, wobei die Schritte des Bewegens der Düse (152) zwischen der ausgefahrenen und eingefahrenen Position durch einen Antriebsmotor (95) eines Antriebsmechanismus bewirkt werden.

24. Verfahren gemäß Anspruch 23, ferner umfassend den Schritt des Betreibens des Antriebsmechanismus, um die Düse (152) zwischen der ausgefahrenen Position und einer Zwischenposition benachbart zu der eingefahrenen Position hin- und herzubewegen, wobei die Zwischenposition außerhalb des Gehäuses (60) liegt.

25. Verfahren gemäß einem der Ansprüche 18 bis 24, ferner umfassend den Schritt des Ablassens der Reinigungsflüssigkeit aus der Ablaufkammer (170) zu einem abnehmbaren Auffangbehälter in Fluidverbindung mit dem Ablaufdurchgang.

## Revendications

1. Dispositif générateur de brouillard comprenant :
un boîtier (60) ;
une buse (152) située à l'intérieur du boîtier (60), la buse pouvant être déplacée entre une position rétractée à l'intérieur du boîtier (60) et une position déployée à l'extérieur du boîtier (60) ;
une chambre de drainage (170) définie entre le boîtier (60) et la buse (152) lorsque la buse est dans la position rétractée ; et
un passage de drainage assurant une communication fluidique à l'écart de la chambre de drainage (170) ;
**caractérisé en ce que** la buse (152) comprend :
une entrée de buse (153) pouvant être raccordée à une source de fluide d'entraînement, une sortie de buse (157) et une gorge de buse (155) entre l'entrée de buse (153) et la sortie de buse (157), la gorge de buse (155) ayant une zone en section transversale qui est inférieure à celle de l'entrée de buse (153) et de la sortie de buse (157) ; et
une chambre de liquide de traitement (145) ayant une entrée pouvant être raccordée à une source de liquide de traitement et une sortie qui débouche dans la buse (152).

2. Dispositif selon la revendication 1, dans lequel la sortie de la chambre de liquide de traitement débouche dans la buse (152) entre la gorge de buse (155) et la sortie de buse (157).

3. Dispositif selon l'une quelconque des revendications précédentes, comprenant en outre une tête de buse (100) sur laquelle la buse (152) est située, la tête de buse (100) ayant un axe longitudinal (L) et dans lequel la buse (152) s'étend dans une direction sensiblement radiale par rapport à l'axe longitudinal (L).

4. Dispositif selon la revendication 3, dans lequel la buse (152) s'étend circonférentiellement autour de la tête de buse (100) de sorte que la buse couvre un angle de rotation autour de l'axe longitudinal (L).

5. Dispositif selon la revendication 4, dans lequel la buse (152) couvre un angle de rotation de sensiblement 360 degrés autour de l'axe longitudinal (L).

6. Dispositif selon l'une quelconque des revendications 3 à 5, comprenant en outre des première et deuxième surfaces opposées sensiblement planes (148, 150) qui définissent la buse (152) entre elles et dans lequel la sortie de la chambre de liquide de traitement (145) est située sur l'une des première et deuxième surfaces.

7. Dispositif selon la revendication 6, dans lequel la chambre de liquide de traitement (145) et la sortie de chambre sont sensiblement annulaires.

8. Dispositif selon une quelconque revendication précédente, comprenant en outre un mécanisme d'entraînement pour déplacer la buse (152) entre les positions rétractée et déployée, le mécanisme comprenant :
une colonne de support (90) montée de manière coulissante dans le boîtier (60) et ayant la buse (152) fixée à celle-ci ;
une tige filetée d'entraînement (97) ayant un chariot d'entraînement (98) monté de manière rotative sur celle-ci ;
un élément de collier (99) comportant une première partie fixée à la colonne de support (90) et une deuxième partie fixée au chariot d'entraînement (98) de sorte que la rotation de la tige d'entraînement (97) se traduise en un mouvement axial du chariot d'entraînement (98) et de la colonne de support (90) ; et
un moteur d'entraînement (95) conçu pour fournir un mouvement de rotation de la tige d'entraînement (97).

9. Dispositif selon la revendication 8, dans lequel le mécanisme d'entraînement effectue un va-et-vient de la buse (152) entre la position déployée et une position intermédiaire adjacente à la position rétractée, la position intermédiaire étant à l'extérieur du boîtier (60).

10. Dispositif selon une quelconque revendication précédente, comprenant en outre un réservoir de récupération amovible en communication fluidique avec le passage de drainage.

11. Dispositif selon une quelconque revendication précédente, dans lequel la chambre de drainage (170) est une chambre annulaire s'étendant autour de la circonférence de la buse (152) lorsque la buse est dans la position rétractée.

12. Dispositif selon la revendication 11 comprenant en outre un joint d'étanchéité (158) disposé autour de la circonférence de la buse (152), dans lequel lorsque la buse est dans la position rétractée, le joint d'étanchéité (158) forme un joint entre la buse et le boîtier (60) et définit partiellement la chambre de drainage (170).

13. Appareil générateur de brouillard portatif comprenant :
une unité de compresseur (10) comprenant un compresseur, une entrée de compresseur fournissant du fluide d'entraînement au compresseur et une sortie de compresseur à travers laquelle le fluide d'entraînement comprimé sort du compresseur ; et
une unité de pulvérisation (40), comprenant :
un dispositif générateur de brouillard selon l'une quelconque des revendications 1 à 13 ;
un récipient de liquide de traitement (82) et un récipient de liquide de nettoyage (81) en communication fluidique avec la buse (152) par le biais d'une ligne d'alimentation de liquide (80) ;
une entrée de fluide d'entraînement ayant une première extrémité pouvant être raccordé à la sortie de compresseur et une seconde extrémité en communication fluidique avec la buse (152) ; et
une première vanne de régulation (73) qui raccorde sélectivement un des récipients de liquide de traitement et de liquide de nettoyage (82, 81) avec la ligne d'alimentation de liquide (80).

14. Appareil selon la revendication 13, dans lequel l'unité de pulvérisation (40) comprend en outre une pompe pneumatique (77) et une ligne d'alimentation de pompe (75) raccordée à l'entrée de fluide d'entraînement, la pompe entraînée par le fluide d'entraînement pour pomper le liquide de traitement ou de nettoyage par le biais de la ligne d'alimentation de liquide (80) vers la buse.

15. Appareil selon la revendication 14, dans lequel la ligne d'alimentation de pompe (75) inclut une ligne de dérivation (78) et une deuxième vanne de régulation (83) située sur la ligne de dérivation (78), moyennant quoi le fluide d'entraînement peut sélectivement contourner la pompe (77) de façon à purger la ligne d'alimentation de liquide (80) et la buse (152).

16. Appareil selon l'une quelconque des revendications 13 à 15, dans lequel l'unité de pulvérisation (40) est portée sur l'unité de compresseur (10) lorsque l'appareil est transporté.

17. Appareil selon la revendication 16, dans lequel l'unité de compresseur (10) comprend une ou plusieurs pinces (38) qui fixent l'unité de pulvérisation (40) à l'unité de compresseur (10) pendant le transport.

18. Procédé d'actionnement d'un dispositif générateur de brouillard ayant une buse (152) située à l'intérieur d'un boîtier (60), le procédé comprenant les étapes consistant à :
déplacer la buse (152) vers une position déployée à l'extérieur du boîtier (60) ;
fournir un liquide de traitement à la buse ;
pulvériser un brouillard de gouttelettes de liquide de traitement à partir de la buse ;
arrêter l'alimentation du liquide de traitement vers la buse ;
déplacer la buse jusqu'à une position rétractée à l'intérieur du boîtier (60) ;
fournir un liquide de nettoyage à la buse ;
pulvériser du liquide de nettoyage de la buse dans une chambre de drainage (170) définie entre le boîtier (60) et la buse rétractée ; et
vidanger le liquide de nettoyage à l'écart de la chambre de drainage (170) par l'intermédiaire d'un passage de drainage ;
**caractérisé en ce que** la buse (152) a une entrée de buse (153), une sortie de buse (157) et une gorge de buse (155) qui a une zone en section transversale plus petite que l'entrée de buse (153) et la sortie de buse (157) et dans lequel l'étape de pulvérisation du brouillard de gouttelettes de fluide de traitement comprend les étapes consistant à :
raccorder une source de fluide d'entraînement à l'entrée de buse (153) ;
raccorder une source du liquide de traitement à une sortie de liquide de traitement qui débouche dans la buse (152) ; et
accélérer le fluide d'entraînement à travers la gorge de buse (155), de telle sorte que le fluide d'entraînement atomise le liquide de traitement entrant dans la buse à travers la sortie de liquide de traitement.

19. Procédé selon la revendication 18, dans lequel la buse (152) s'étend dans une direction sensiblement radiale par rapport à un axe longitudinal (L) du dispositif et les étapes de pulvérisation des liquides de traitement et de nettoyage comprennent la pulvérisation des liquides respectifs radialement autour de l'axe longitudinal (L).

20. Procédé selon la revendication 19 dans lequel la buse (152) couvre un angle de rotation de sensiblement 360 degrés autour de l'axe longitudinal (L) et les étapes de pulvérisation des liquides de traitement et de nettoyage comprennent la pulvérisation des liquides respectifs sur sensiblement 360 degrés autour de l'axe longitudinal (L).

21. Procédé selon l'une quelconque des revendications 18 à 20, dans lequel les étapes d'alimentation des liquides vers la buse (152) comprennent le pompage des liquides en utilisant une pompe pneumatique (77) qui est entraînée par le fluide d'entraînement depuis la source de fluide d'entraînement.

22. Procédé selon l'une quelconque des revendications 18 à 21, comprenant en outre l'étape de purge de la buse du liquide de traitement et/ou de nettoyage en utilisant un fluide d'entraînement provenant de la source de fluide d'entraînement.

23. Procédé selon l'une quelconque des revendications 18 à 22, dans lequel les étapes de déplacement de la buse (152) entre les positions déployée et rétractée sont effectuées par un moteur d'entraînement (95) d'un mécanisme d'entraînement.

24. Procédé selon la revendication 23, comprenant en outre l'étape consistant à actionner le mécanisme d'entraînement de manière à faire effectuer un va-et-vient à la buse (152) entre la position déployée et une position intermédiaire adjacente à la position rétractée, la position intermédiaire étant à l'extérieur du boîtier (60).

25. Procédé selon l'une quelconque des revendications 18 à 24, comprenant en outre l'étape consistant à drainer le liquide de nettoyage de la chambre de drainage (170) vers un réservoir de récupération amovible en communication fluidique avec le passage de drainage.
